# EUROPEAN PATENT APPLICATION

(11) **EP 4 134 447 A1**
(43) Date of publication of application: **15.02.2023**
(21) Application number: 21194900.3
(22) Date of filing: 03.09.2021
(51) Int. Cl.: C12Q 1/6883, C12Q 1/689

(54) **METHOD FOR THE PROGNOSIS OF SHEEP FOOTROT**

(30) Priority: 13.08.2021 PT 2021117396
(71) Applicant: ACOS - Associação de Agricultores do Sul, 7800-453 Beja (PT); Cebal - Centro De Biotecnologia Agrícola E Agro-Alimentar Do Alentejo, 7800-309 Beja (PT); Universidade De Évora, 7004-516 Évora (PT); INIAV - Instituto Nacional de Investigação Agrária E Veterinária, 2780-157 Oeiras (PT)
(72) Inventor: PEREIRA MATOS, CLAUDINO ANTÓNIO, 7800-453 BEJA (PT); COSTA DO AMARAL RAMOS, ANTÓNIO MARCOS, 7800-309 BEJA (PT); USIÉ CHIMENOS, ANA ISABEL, 7800-309 BEJA (PT); BRANCO GASPA, DANIEL FILIPER, 7800-309 BEJA (PT); GODINHO VIEIRA MONTEIRO, MARIA HELENA, 7800-453 BEJA (PT); FIALHO LEÃO, CÉLIA CRISTINA, 2780-157 OEIRAS (PT); PIMENTEL CAROLINO, RENATO NUNO, 2005-048 VALE DE SANTARÉM (PT); DA SILVA BRANCO, SANDRA MARIA, 7002-554 ÉVORA (PT); NETO PADRE, LUDOVINA, 7002-554 ÉVORA (PT); VARELA BETTENCOURT HENRIQUES, ELISA MARIA, 7002-554 ÉVORA (PT); DE SOUSA HENRIQUES, PEDRO DAMIÃO, 7000-803 Évora (PT)
(74) Representative: Neves, Ana

(57) **Abstract**

The present patent application discloses a method for prognosis and characterization of sheep footrot based on the use of data produced using state-of-the-art sequencing techniques to characterize the composition of the microbiome associated with the foot rot in sheep. This characterization is done qualitatively, by identifying the species of bacteria present in the microbiome of each sample collected, but also quantitatively, as the abundance of each species, in each sample analyzed, is also determined. Subsequently, in situations where the observed score for the footrot is available for each animal, it is possible to identify the most important differences between animals not affected by footrot and those more affected by this disease.

## Description

### Technical field

This application relates to a method for the prognosis of footrot in sheep.

### Background art

Sheep footrot is one of the main causes of lameness in sheep and represents a major animal welfare problem due to the painful nature of the lesions. This disease is caused by infection with *Dichelobacter nodosus* (*D. nodosus*)*,* causing painful inflammation, necrotizing lesions of the interdigital skin, characteristic odor, and hoof detachment. Sheep footrot is a multifactorial disease and its severity depends on the breed of sheep, farm management, environmental factors, the presence of co-infecting bacteria, and the virulence of the *D. nodosus* strains involved. In addition, the bacterium *Fusobacterium necrophorum* (*F. necrophorum*) has also been suggested as a secondary pathogen in the development of the disease and probably in increasing its severity. The synergistic relationship between *F. necrophorum* and *D. nodosus* is not yet fully explained, but it appears that their combined action is involved in the development and severity of footrot.

Although the involvement of these two bacterial species is well established, the universe of bacterial species present is much more comprehensive, and no information is available on the composition and abundance of the remaining species present in this microbiome. For this reason, it is important to have available a method that can accurately decipher the bacterial species present, as well as their abundance, so that the main differences in the microbiome between animals with contrasting phenotypes for the degree of footrot development can be evaluated.

### Summary

The present patent application relates to a method for prognosis of footrot in sheep.

The method for prognosis of sheep footrot disclosed herein comprises the following steps:
- Preparation of the skin biopsy sample from an animal to be evaluated;
- Detecting the microbiome signature present in the biopsy sample comprising the genera
   I) *Mycoplasma, Campylobacter, Streptococcus, Clostridium, Arcobacter, Fusobacterium (10 species), Capnocytophaga, Treponema, Leptotrichia,* and *Vibrio;* and/or
   II) *Streptomyces, Pseudomonas, Mycolicibacterium, Deinococcus, Brevundimonas, Micromonospora, Rhodococcus, Staphylococcus, Mycobacterium* and *Gordonia;*
   Concluding on footrot development probability in said animal, wherein an increase of abundance of the genera in I) over the genera in II) as detected in said sample is indicative for footrot.

### Detailed Description

The present patent application discloses a method for prognosis of sheep footrot based on the use of data produced using state-of-the-art sequencing techniques to characterize the composition of the microbiome associated with the foot rot in sheep. This characterization is done qualitatively, by identifying the species of bacteria present in the microbiome of each sample collected, but also quantitatively, as the abundance of each species, in each sample analyzed, is also determined. This method thus allows a detailed characterization of the microbiome present in each animal. Subsequently, in situations where the observed score for the footrot is available for each animal, it is possible to identify the most important differences between animals not affected by footrot and those more affected by this disease.

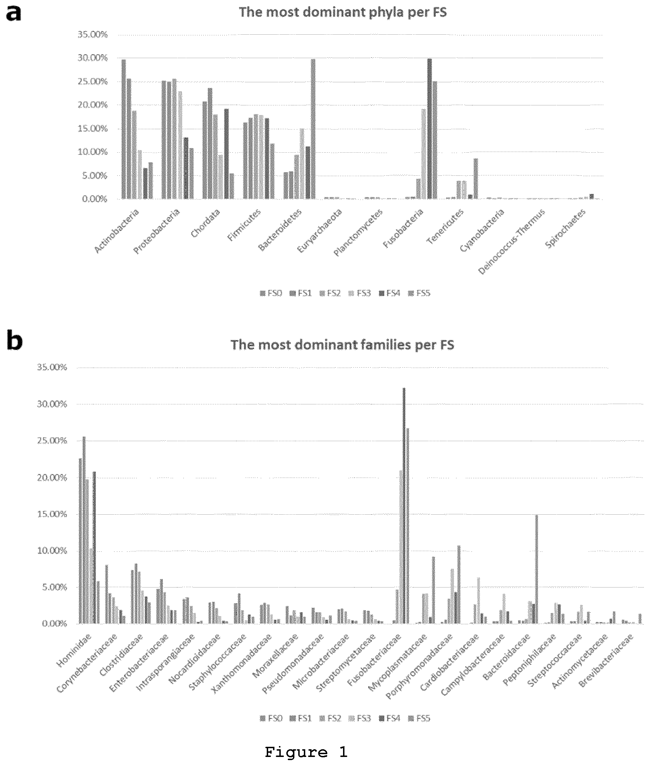

According to the method disclosed herein the sequencing data is first processed to remove the lowest quality sequences. A taxonomic classification is then performed to determine the number of species present, followed by an estimate of the abundance of each of these species in each of the animals analyzed. In situations where microbiomes have been collected from animals with contrasting scores for the footrot, a statistical analysis is performed to determine the number of species with differential abundance between animals affected and unaffected by the footrot.
In the data analyzed to develop this technology, 213 animals were included. In each animal a skin/tissue biopsy was collected from one of the legs, biological material from which DNA extraction was performed. On average, for each animal 545,000 sequences were used in the analyses. The results of the taxonomic classification revealed the presence of 64 phyla and 505 families of bacteria. For each footrot score (FS), at the phylum level the results obtained were similar. The most abundant phylum was *Actinobacteria* in samples with FS0 and FS1 scores (29.7% and 25.6%, respectively), *Proteobacteria* in samples with FS2 and FS3 scores (25.6% and 22.8%, respectively), *Fusobacteria* in samples with FS4 score (29.9%), and Bacteroides in samples with FS5 score (29.8%). Regarding the families identified, *Hominidae* was the most dominant in samples with FS0, FS1 and FS2 (22.6%, 25.6% and 19.7%, respectively), and the second most dominant in samples with FS3 and FS4 (10.3% and 20.80%, respectively), where the most dominant family was *Fusobacteriaceae* (21.0% and 32.2%, respectively). In the samples with FS5, the most dominant family was also *Fusobacteriaceae* (26.7%), followed by *Bacteroidaceae* (14.9%). These results are illustrated in Figure 1.

A new classification of all samples was made in order to define groups of animals considered as not affected by the footrot (scores FS0 and FS1), and another group of animals affected by the footrot (scores FS2, FS3 and FS4). Next, a differential abundance analysis was performed between the 2 groups, which revealed 656 species with significant differences in abundance between the groups (169 species were significantly more abundant in the infection-free samples and 487 in the infected samples). The top 10 genera that were significantly more abundant in footrot infection samples were Mycoplasma (47 species), Campylobacter (37 species), Streptococcus (35 species), Clostridium (27 species), Arcobacter (22 species), Fusobacterium (10 species), Capnocytophaga (10 species), Treponema (8 species), Leptotrichia (8 species), and Vibrio (7 species). Regarding the genera found significantly more abundant in the infection-free samples, the top 10 were Streptomyces (44 species), Pseudomonas (13 species), Mycolicibacterium (9 species), Deinococcus (7 species), Brevundimonas (7 species), Micromonospora (6 species), Rhodococcus (6 species), Staphylococcus (5 species), Mycobacterium (5 species) and Gordonia (3 species).

The method for prognosis of sheep footrot disclosed herein comprises the following steps:
- Preparation of the skin biopsy sample from an animal to be evaluated;
- Detecting the microbiome signature present in the biopsy sample comprising the genera
   III) *Mycoplasma, Campylobacter, Streptococcus, Clostridium, Arcobacter, Fusobacterium (10 species), Capnocytophaga, Treponema, Leptotrichia,* and *Vibrio;* and/or
   IV) *Streptomyces, Pseudomonas, Mycolicibacterium, Deinococcus, Brevundimonas, Micromonospora, Rhodococcus, Staphylococcus, Mycobacterium* and *Gordonia;*
- Concluding on footrot development probability in said animal, wherein an increase of abundance of the genera in I) over the genera in II) as detected in said sample is indicative for footrot.

### Brief description of drawings

For easier understanding of this application, figures are attached in the annex that represent the preferred forms of implementation which nevertheless are not intended to limit the technique disclosed herein.

Figure 1 shows the most represented phyla and families in samples with different scores for the footrot.

## Claims

1. Method for prognosis of sheep footrot comprising the following steps:
- Preparation of the skin biopsy sample from an animal to be evaluated;
- Detecting the microbiome signature present in the biopsy sample comprising the genera
V) *Mycoplasma, Campylobacter, Streptococcus, Clostridium, Arcobacter, Fusobacterium (10 species), Capnocytophaga, Treponema, Leptotrichia,* and *Vibrio;* and/or
VI) *Streptomyces, Pseudomonas, Mycolicibacterium, Deinococcus, Brevundimonas, Micromonospora, Rhodococcus, Staphylococcus, Mycobacterium* and *Gordonia;*
Concluding on footrot development probability in said animal, wherein an increase of abundance of the genera in I) over the genera in II) as detected in said sample is indicative for footrot.
